(19) Europäisches Patentamt
European Patent Office
Office européen des brevets

(11) Publication number: **0 456 504 A2**

# (12) EUROPEAN PATENT APPLICATION

(21) Application number: **91304206.5**

(51) Int. Cl.[5]: **C07D 213/803**

(22) Date of filing: **09.05.91**

(30) Priority: **11.05.90 US 522285**

(43) Date of publication of application: **13.11.91 Bulletin 91/46**

(84) Designated Contracting States: **AT BE CH DE DK ES FR GB IT LI NL SE**

(71) Applicant: **HOECHST CELANESE CORPORATION**
**Route 202-206 North**
**Somerville, New Jersey (US)**

(72) Inventor: **Larkin, Donald R.**
**442 Williamson**
**Corpus Christi, Texas 78411 (US)**
Inventor: **Elango, Varadaraj**
**4175 Crenshaw Drive**
**Corpus Christi, Texas 78413 (US)**
Inventor: **Fritch, John R.**
**4334 Five Points Road**
**Corpus Christi, Texas 78410 (US)**
Inventor: **Mueller, Werner H.**
**13825 Eaglesnest Bay**
**Corpus Christi, Texas (US)**
Inventor: **Gupton, B. Franklin**
**1012 Camino Real South**
**Virginia Beach, Virginia (US)**

(74) Representative: **De Minvielle-Devaux, Ian Benedict Peter et al**
**CARPMAELS & RANSFORD 43, Bloomsbury Square**
**London WC1A 2RA (GB)**

## (54) Process for preparing pyridinecarboxylic acid derivatives.

(57) Pyridine carboxylic acid derivatives are prepared by reacting an oxime with an alkene at neutral or weakly acid conditions.

EP 0 456 504 A2

## BACKGROUND OF THE INVENTION

Literature methods for preparing 5,6-dialkyl and 5-alkyl-6-arylpyridine-2,3-dicarboxylic acids and esters are limited and often require oxidation of alkyl or aryl substituents at positions 2 and 3 in order to obtain diacids. Recently, there has been disclosed a method for the preparation of substituted and disubstituted pyridine-2,3-dicarboxylic acid esters and 2-alkylnicotinates utilizing α-halo-β-ketoesters and α,β-unsaturated aldehydes or ketones in the presence of an ammonium salt. The use of α-halo-β-ketoesters is not desired due to the fact that such materials are usually costly and unstable.

U. S. Patent 4,723,011 discloses preparation of substituted and disubstituted pyridine-2,3-dicarboxylic acid esters by the reaction of an α-halo-β-ketoester such as chloro-diethyloxalacetate (chloro-DOX) and an α,β-unsaturated aldehyde or ketone such as 2-ethylacrolein in the presence of at least 2 molar equivalents of an ammonium salt in order to produce the desired compounds.

U. S. Patent 4,816,588 discloses and claims a process for preparing pyridine-2,3-dicarboxylic acids by the oxidation of 8-substituted quinolines.

European Patent Application No. 274,379, published July 13, 1988, discloses two processes for producing pyridine-2,3-dicarboxylic acid compounds. One process seems similar to that previously described in U. S. Patent 4,723,011 and the other process involves reacting an α,β-unsaturated aldehyde or ketone with various aminomaleates or aminofumarates such as diethyl aminomaleate.

European Patent Application No. 299,362, published January 18, 1989, also discloses the same reaction.

European Patent Application No. 308,084, published March 22, 1989, discloses the preparation of pyridine-2,3-dicarboxylic acid compounds by reacting an oxime with an α-halo-diester followed by in-situ dehydration.

The commercial importance of the pyridine dicarboxylic acid derivatives, particularly as useful intermediates for the preparation of herbicidal 2-(2-imidazolin-2-yl)nicotinic acids, esters and salts, makes any improvement in their processes for production of tremendous potential economic significance.

## SUMMARY OF THE INVENTION

It has now been found that pyridinedicarboxylic acid derivative of Formula I

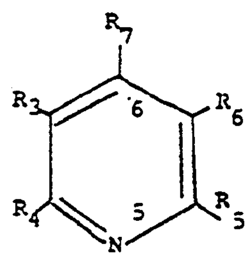

can be prepared by reacting an oxime of the formula:

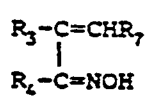

II

with an alkene of formula III or IIIA

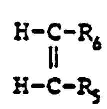

III

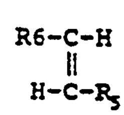

IIIA

wherein $R_3$ hydrogen, halogen, $C_1$-$C_6$ straight or branched alkyl, alkenyl, phenyl, or substituted-phenyl; $R_4$ and $R_7$ are each independently hydrogen, $C_1$-$C_6$ straight or branched alkyl, alkenyl, phenyl, or substituted-phenyl;

$R_3$ and $R_4$ taken together can be $-(CH_2)_{3-10}$; $R_5$ and $R_6$ are each independently H, COZ, or CN, provided that they are not both H; or $R_5$ and $R_6$ together are $-CO-NR_{10}-CO$; Z is $OR_8$ or $NR_8R_9$; $R_8$ and $R_9$ are each independently H or alkyl (preferably $C_1$-$C_6$ straight or branched alkyl) or aryl; or $R_8$ and $R_9$ together with the nitrogen atom form aliphatic or aromatic heterocyclic structures and $R_{10}$ is hydrogen, alkyl (preferably $C_1$-$C_6$ straight or branched alkyl), aryl, hydroxy or an alkoxy of 1-6 carbon atoms.

The expression "substituted phenyl" is intended to mean phenyl substituted in one or more positions with substituents such as alkyl; halogen (bromine, chlorine, fluorine or iodine); hydroxy; alkoxy of 1-7 carbon atoms; cyano; nitro; or amino.

The reaction exhibits improved efficiency when carried out under neutral or, preferably, weakly acid conditions at temperatures ranging from 80°-250°C, preferably 130°-145°C. The present reaction system is anhydrous, and thus it is difficult to measure acidity directly; however, if water were present, the pH would range between 2 and 7 and preferably between 3 and 4.

The mole ratio of the oximes of Formula II to the alkenes of formula III or IIIa is not critical and can range from about 1:3 to 3:1. It is preferred to use approximately 1:1 molar ratios.

Anhydrous $SiO_2$ is a useful catalyst. The amount of catalyst used can range from 1.0 to 200 mole %, preferably 1 to 100 mole % with respect to the oxime.

EXAMPLE 1

Preparation of Crude Oxime of 2-Ethylacrolein (2)

A mixture of 126.90 g (1.50 mole) of 2-ethylacrolein, 111.66 g (1.6 mole) of hydroxylamine hydrochloride, and 100 mL of water was placed in a 1-L round bottom flask equipped with a reflux condenser, magnetic stirrer, thermometer and dropping funnel. A solution of 110.69 g (1.5 equivalents) of potassium carbonate in 100 mL of water was added to the stirred reaction mixture over a two minute period. The mixture was stirred for about 40 minutes. The temperature rose to 44°C. The organic phase, crude 2-ethylacrolein oxime, was separated and found to weigh 147 g. (The theoretical yield is 148.5 g).

EXAMPLE 2

Driving of Crude 2-Ethylacrolein Oxime

A solution of 70.7 g of crude oxime of 2-ethylacrolein of Example 1 in 400 mL of benzene was refluxed for 3 hours in a 1-L flask equipped with Dean-Stark trap and reflux condenser. Over a 3-hour period, 4 mL of water removed. The benzene was then removed under a vacuum using steam heating to leave 68.4 g of dried oxime.

EXAMPLE 3

Distillation of Dried 2-Ethylacrolein Oxime

To prevent its dehydration to the nitrile or polymerization, the dried oxime of 2-ethylacrolein from Example 2 was distilled, with the purified fraction collected at an overhead temperature of 25°C and at a pressure of 0.2 mm Hg absolute. Initially, the pot temperature was 45°C; during the distillation, it slowly rose to 110°C.

EXAMPLES 4-53

Condensation of 2-Ethylacrolein Oxime with Diethyl Maleate (3)

Stock solutions were prepared by mixing a G. C. internal standard (0.96 g) with 5.00 g (0.050 mole) of oxime of 2-ethylacrolein and 8.010 g (0.0465 mole) of diethyl maleate.

Portions (1.00 g) of the stock solution were mixed with additives and sealed into Reactivials. Reactivials were heated for 2 to 3 hours, cooled, and sampled.

The sample was analyzed by gas liquid chromatography. This process was then repeated 2 to 5 times depending on the reaction temperature chosen. Often, the Reactivials were heated overnight before taking final samples. During the reactions, some diethyl maleate isomerized to diethyl fumarate. Depending on the temperature, normally 80 to 95% of the diethyl maleate and diethyl fumarate were consumed in 6 hours. Results are summarized below. Both the average and the highest observed efficiencies (Eff.) to diethyl 5-ethylpyridine-2,3-dicarboxylate (Et 5-EPDC) observed are shown in the following table. Efficiency as used herein is defined as follows:

Eff. = moles of product per 100 moles of reacted diethyl maleate.

Reactions with Crude Oxime with p-Xylene as Standard

| Example | Additive | Wt.% or Mole % of Add.* | Temp.°C | Time Hours | Aver. Eff. | High Eff. |
|---|---|---|---|---|---|---|
| 4 | None | | 113 | 4.5 | 0.0 | 0.0 |
| 5 | None | | 132 | 6.0 | 2.8 | 2.8 |
| 6 | None | | 145 | 9.5 | | 21.1 |
| 7 | Nitrobenzene and Pd/C | 1.8 | 142 | 10.5 | 21.4 | 21.6 |
| 8 | None | | 150 | 3 | | 9.4 |
| 9 | None | | 175 | 3 | | 7.2 |
| 10 | None | | 200 | 2 | | 4.3 |
| 11 | None | | 250 | 2 | | 2.2 |
| 12 | $BF_3Et_2O$ | 0.10 | 145 | 10 | 2.0 | 2.0 |
| 13 | #56 $SiO_2$ | 15.3 | 145 | 12 | | 17.0 |
| 14 | #56 $SiO_2$ | 3.2 | 145 | 10 | 4.4 | 6.0 |
| 15 | #56 $SiO_2$ | 3.2 | 135 | 8 | 3.0 | 4.5 |
| 16 | #56 $SiO_2$ | 3.2 | 135 | 8 | 4.2 | 4.4 |

* Weight % applies to $SiO_2$ Additive and mole % applies to all other Additives.

Reactions with Crude Oxime with n-Hexadecane as Standard

| Example | Additive | Wt.% of Add. | Temp.°C | Time Hours | Aver. Eff. | High Eff. |
|---|---|---|---|---|---|---|
| 17 | None | | 130 | 17 | 20.5 | 20.5 |
| 18 | $SiO_2$ | 3.64 | 130 | 25 | 20.3 | 20.9 |
| 19 | $SiO_2$ | 3.92 | 130 | 25 | 21.0 | 21.8 |
| 20 | $SiO_2$ | 7.92 | 130 | 25 | 21.3 | 24.2 |
| 21 | $SiO_2$ | 8.00 | 130 | 25 | 21.1 | 22.4 |
| 22 | $SiO_2$ | 30.9 | 130 | 11 | 24.1 | 24.1 |
| 23 | $SiO_2$ | 56.9 | 130 | 11 | 25.1 | 25.5 |
| 24 | None | | 140 | 9 | 21.4 | 21.7 |

Reactions with Dried, Crude Oxime with n-Hexadecane as Standard

| Example | Additive | Wt. % of Add. | Temp.°C | Time Hours | Aver. Eff. | High Eff. |
|---|---|---|---|---|---|---|
| 25 | None | | 130 | 11 | 18.8 | 19.9 |
| 26 | $SiO_2$ | 33.8 | 130 | 11 | 28.1 | 28.8 |
| 27 | None | | 140 | 9 | 17.7 | 17.8 |
| 28 | $SiO_2$ | 30.9 | 140 | 9 | 23.1 | 23.8 |
| 29 | $SiO_2$ | 55.5 | 140 | 9 | 26.0 | 26.1 |

Reactions with Dried, Purified Oxime With n-Hexadecane as Standard

| Example | Additive | Wt. % or Mole % of Add.* | Temp. °C | Time Hours | Aver. Eff. | High Eff. |
|---|---|---|---|---|---|---|
| 30 | None | | 140 | 80 | 3.2 | 7.4 |
| 31 | None | | 138 | 8 | 5.5 | 6.7 |
| 32 | $SiO_2$ | 29.5 | 140 | 80 | 8.8 | 10.4 |
| 33 | $K_2CO_3$ | 0.28 | 138 | 8 | | trace |
| 34 | $K_2CO_3$ | 0.31 | 138 | 8 | 16.0 | 16.5 |
| | $NH_2OH.HCl$ | 0.27 | | | | |
| 35 | $NH_2OH.HCl$ | 0.27 | 138 | 8 | 17.9 | 21.5 |
| 36 | $NH_4$sulfam | 0.15 | 136 | 7.5 | 1.4 | 1.4 |
| 37 | $NH_4$sulfam | 0.06 | 136 | 7.5 | 15.4 | 20.6 |
| | $H_2O$ | | | | | |
| 38 | $NH_4HCO_3$ | 1.0 | 25 | 17 | 0.0 | 0.0 |
| | $NH_2OH.HCl$ | 1.0 | | | | |
| | EtOH | | | | | |
| 39 | $NH_4HCO_3$ | 1.0 | 110 | 4.25 | 11.0 | 12.3 |
| | $NH_2OH.HCl$ | 1.0 | | | | |
| 40 | $NH_4HCO_3$ | 1.0 | 25 | 17 | 0.0 | 0.0 |
| | $NH_2OH.HCl$ | 1.0 | | | | |
| 41 | $NH_4HCO_3$ | 1.0 | 110 | 4.25 | 5.8 | 7.9 |
| | $NH_2OH.HCl$ | 1.0 | | | | |
| | EtOH | | | | | |
| 42 | $K_2CO_3$ | 1.0 | 110 | 6 | 2.2 | 3.3 |
| | $NH_2OH.HCl$ | 1.0 | | | | |

* Weight % applies to $SiO_2$ Additive and mole % applies to all other Additives.

Reactions With Purified Oxime Containing 0.28 Wt. % Phenothiazir (a Polymerization Inhibitor) with n-Hexadecane as Standard

| Example | Additive | Wt.% or Mole % of Add.* | Temp.°C | Time Hours | Aver. Eff. | High Eff. |
|---|---|---|---|---|---|---|
| 43 | Aq.$NH_2OH$ | 0.58 | 115 | 4 | 15.8 | 19.9 |
| | Mn | $6 \times 10^{-4}$ | | | | |
| 44 | Aq.$NH_2OH$ | 0.58 | 115 | 4 | 16.2 | 16.3 |
| | Mn | $6 \times 10^{-4}$ | | | | |
| | Cu | $3 \times 10^{-3}$ | | | | |
| 45 | Aq.$NH_2OH$ | 0.43 | 130 | 4 | 21.7 | 23.5 |
| | Mn | $6 \times 10^{-4}$ | | | | |
| | $SiO_2$ | 34.7 | | | | |
| 46 | Aq.$NH_2OH$ | 0.42 | 130 | 4 | 19.8 | 21.2 |
| | Mn | $6 \times 10^{-4}$ | | | | |
| | $SiO_2$ | 33 | | | | |
| 47 | p-xylene | 45 | 128 | 5.5 | | 17.5 |
| | $NH_2OH$ | trace | | | | |
| | $H_2O$ | trace | | | | |
| | $SiO_2$ | 43 | | | | |
| 48 | p-xylene | 45 | 128 | 5.5 | 23.8 | 24.7 |
| | $NH_2OH$ | trace | | | | |
| | $H_2O$ | trace | | | | |
| | $SiO_2$ | 81 | | | | |
| 49 | p-xylene | 45 | 128 | 5.5 | | 0.0 |
| | $NH_2OH$ | trace | | | | |
| | $H_2O$ | trace | | | | |
| 50 | p-xylene | 45 | 128 | 5.5 | | 0.0 |
| | $NH_2OH$ | trace | | | | |
| | $H_2O$ | trace | | | | |
| 51 | $NH_2OH$.AcOH | 0.34 | 128 | 9.5 | 23.6 | 26.3 |
| | nitrobenzene | 1.1 | | | | |
| | $SiO_2$ | 17.7 | | | | |
| | Pd/C | 0.05 | | | | |
| 52 | $NH_2OH$.AcOH | 1.08 | 128 | 9.5 | 26.2 | 26.3 |
| | nitrobenzene | 1.09 | | | | |
| | $SiO_2$ | 17.6 | | | | |
| | Pd/C | 0.07 | | | | |
| 53 | Sulfur | 10 | 133 | 6 | | 5.8 |

* Weight % applies to $SiO_2$ Additive and mole % applies to all other Additives.

From the above Examples it can be seen that simply heating a solution of the unpurified oxime of 2-ethyl acrolein (2) and diethyl maleate (3) to 130 to 140°C was shown to give 15 to 25% yields of Et 5-EPDC (1) (Examples 4-29). Only traces of the diethyl ester of 5-ethyldihydropyridine-1,2-dicarboxylic acid (4) were detected.

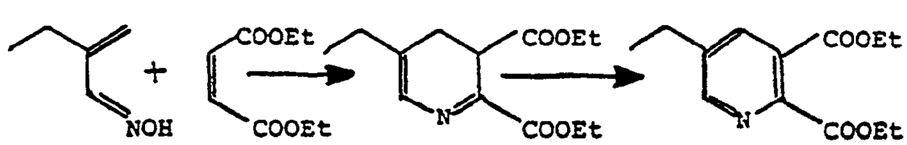

2 3 4 1

Replacing the crude oxime with purified oxime gave 5% or less of (1) (Examples 30-31). It was necessary to spike the purified oxime with hydroxylamine HCl or other acid to get Et 5-EPDC (1) yields back into the 15-25% range (Examples 34, 35). Under neutral or weakly acidic conditions, we assume that the oxime adds across the double bond of the diethyl maleate to form the Michael adduct (5). Ring closure and dehydration to (4) followed by dehydrogenation then yields (1). In the presence of a weak base such as $K_2CO_3$, the main product

of the reaction of (2) and (3) is the other Michael adduct (6).

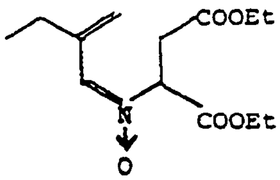

5

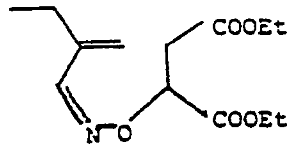

6

In Example 33, with $K_2CO_3$as catalyst, the main product (38% yield) was the alternate adduct (6); only traces of Et 5-EPDC (1) were formed. In Example 34, in which both $K_2CO_3$ and $NH_2OH.HCl$ were present at the same time as undissolved solids, the main product was EPDC (16%) and 3.5% of (6) was formed. In Example 35, in which only $NH_2OH.HCl$ was present, the only detectable product was Et 5-EPDC (1); no (6) was formed.

## EXAMPLE 54

### Preparation of 0-[2-(Diethyl Succinyl)] Derivative of Oxime of 2-Ethylacrolein

A solution containing 865 mg (5 mmole) of diethyl maleate, 540 mg (5.45 mmole) of 2-ethylacrolein oxime, 92.2 mg of n-hexadecane (GC internal standard), and 4.6 mg of phenothiazine (polymerization inhibitor) was mixed with 107.2 mg of finely powdered potassium carbonate and heated to 110°C in a sealed Reactivial. Samples were taken periodically for analysis. The course of the reaction is shown below. As the reaction proceeded, some of the diethyl maleate (3) was converted to diethyl fumarate (3B). In the presence of the weak base, potassium carbonate, little or no Et 5-EPDC (1) was formed and the efficiency to (6) was 87%.

Compounds (MMoles)

| Time Hours | Oxime (2) | Maleate (3) | Fumarate | Product (6) |
|---|---|---|---|---|
| 0.25 | 5.49 | 4.45 | 0.37 | 0.73 |
| 0.50 | 5.26 | 4.17 | 0.40 | 1.03 |
| 0.75 | 4.97 | 3.93 | 0.40 | 1.21 |
| 1.00 | 4.07 | 3.59 | 0.28 | 1.31 |
| 2.00 | 2.98 | 2.57 | 0.42 | 1.66 |
| 3.00 | 1.89 | 2.28 | 0.40 | 1.93 |
| 4.00 | 2.33 | 2.00 | 0.64 | 2.19 |
| 5.00 | 2.10 | 1.63 | 0.62 | 2.39 |

The mass and NMR spectra of (6) agree with the assigned structure.

## EXAMPLE 55

### Reaction of Diethyl Maleate with 2-Ethylacrolein Methoxyoxime

The methoxyoxime of 2-ethylacrolein was assumed to be 70% pure (from area normalized GC analysis). A mixture of 1.752 g (0.010 mole) of diethyl maleate, 1.614 g (0.010 mole assuming 70% purity) of the crude methoxyoxime of 2-ethylacrolein and 0.2116 g of p-xylene (GC internal standard) was sealed in a thick-walled glass vial which was placed in a heating block and heated to 134-136°C. The vial was equipped with a sampling valve with a silicone septum so that samples could be removed with a syringe for GC analysis. In this case, it was not possible to remove periodic samples for analysis because of pressure build-up; however, less than 1% yield was achieved. Yield is defined as conversion x efficiency.

## EXAMPLES 56-63

The procedure of Example 24 is repeated except that the following alkenes and oximes are used:

Alkenes

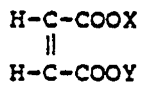

Oximes

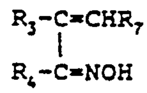

| | X | Y | $R_3$ | $R_4$ | $R_7$ |
|---|---|---|---|---|---|
| Example 56 | methyl | propyl | H | H | phenyl |
| Example 57 | propyl | propyl | phenyl | ethyl | methyl |
| Example 58 | butyl | butyl | ethyl | methyl | H |
| Example 59 | ethyl | ethyl | methyl | H | H |
| Example 60 | ethyl | ethyl | H | methyl | H |
| Example 61 | ethyl | ethyl | H | H | methyl |
| Example 62 | ethyl | ethyl | ----$(CH_2)_3$---- | | H |
| Example 63 | ethyl | ethyl | ----$(CH_2)_4$---- | | H |

## Claims

1. A process for the preparation of pyridine carboxylic acid derivatives of the formula

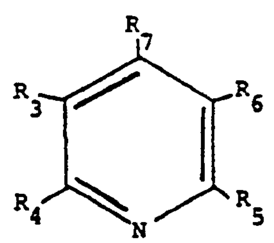

I

wherein $R_3$ is hydrogen, halogen, $C_1$-$C_6$ straight or branched alkyl, alkenyl, phenyl, or substituted-phenyl; $R_4$ and $R_7$ are each independently hydrogen, $C_1$-$C_6$ straight or branched alkyl, alkenyl, phenyl, or substituted phenyl; $R_3$ and $R_4$ taken together can be $-(CH_2)-_{3-10}$; and $R_5$ and $R_6$ are each independently H, COZ, or CN, provided that they are not both H; or $R_5$ and $R_6$ together are $-CO-NR_{10}-CO$; Z is $OR_8$ or $NR_8R_9$; $R_8$ and $R_9$ are each independently H or alkyl or aryl; or $R_8$ and $R_9$ together with the nitrogen atom form an aliphatic or aromatic heterocyclic structure and $R_{10}$ is hydrogen, alkyl, aryl, hydroxy or an alkoxy of 1-6 carbon atoms; comprising reacting an oxime of formula II:

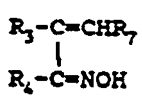

II

wherein $R_3$, $R_4$ and $R_7$ are defined above with an alkene of formula III or IIIA

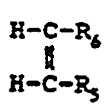

III

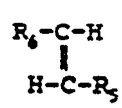

IIIA

wherein $R_5$ and $R_6$ are as described in Formula I at neutral or slightly acid conditions and in a temperature range of 80-250°C.

2. A process according to claim 1 wherein the temperature is 130-145°C.

3. The process of claim 1 wherein the reaction is carried out under conditions which are weakly acidic to neutral, equivalent to a pH of 2 to 7.

4. The process of claim 1 wherein the reaction is carried out under conditions wherein the acidity is equivalent to a pH of 3 to 5.

5. A process according to claim 1 wherein the oxime is 2-ethylacrolein oxime.

6. A process according to claim 5 wherein the alkene is diethyl maleate.

7. A process according to claim 1 for the preparation of diethyl 5-ethylpyridine-2,3-dicarboxylate.

8. A process according to claim 1 for the preparation of diethyl 5-methylpyridine-2,3-dicarboxylate.

9. A process according to claim 1 for the preparation of diethyl 6-methylpyridine-2,3-dicarboxylate.

10. A process according to claim 1 for the preparation of diethyl 4-methylpyridine-2,3-dicarboxylate.

11. A process according to claim 1 for the preparation of diethyl pyridine-2,3-dicarboxylate.